# EUROPEAN PATENT APPLICATION

(11) **EP 4 324 498 A1**
(43) Date of publication of application: **21.02.2024**
(21) Application number: 23192087.7
(22) Date of filing: 18.08.2023
(51) Int. Cl.: A61M 5/142, A61M 5/145

(54) **MULTI-START LEADSCREW FOR VARIABLE FILLING AND PERFORMANCE**

(30) Priority: 19.08.2022 US 202263371879 P
(71) Applicant: Insulet Corporation, Acton MA 01720 (US)
(72) Inventor: MCGAHREN, Lucas, Boston (US); BARNES, Jeffrey, Medford (US); CARDINALI, Steven, Tewksbury (US); ALLIS, Daniel, Boxford (US)
(74) Representative: Peterreins Schley

(57) **Abstract**

A device for delivering fluid to a user may include a reservoir that holds the fluid and a plunger received in the reservoir. As the plunger is driven into the reservoir, the fluid is driven out; similarly, as the reservoir is filled, the plunger is driven out of the reservoir. The plunger may be moved into or out of the reservoir by the action of a lead screw. The lead screw may include multiple starts.

## Description

### BACKGROUND

In fluid delivery devices, a fluid may be held in a reservoir until being dispensed. For instance, fluid delivery devices may include delivery devices for therapeutic fluids, such as insulin or therapeutic drugs. The fluid may be dispensed from the reservoir using a plunger that advances into the reservoir to displace the fluid.

The plunger may be driven by a lead screw. As the lead screw rotates, it may advance a plunger, or an attachment, linearly along the length of the lead screw. The attachment may be fixed to the plunger, so that rotation of the lead screw also advances the plunger.

A lead screw includes a thread, which can be represented as a helical ridge wrapped around the screw. In cross section, the thread extends away from the body of the screw by a certain distance. The outer edge of the thread is referred to as the crest, and the innermost point is referred to as the root. The diameter of the screw as measured at the inner points corresponding to the root of the thread is the minor diameter of the screw, whereas the diameter at the crests is the screw's major diameter.

The distance between adjacent crests in the screw is referred to as the screw's pitch. A similar concept is referred to as the screw's lead - the lead of the screw represents the distance along the screw's axis that is covered by one complete 360 degree rotation of the screw's thread.

Most screws include only a single ridge wrapped around a cylinder or cone. Such screws are referred to as "single-start" or "single lead" screws. In a single-start screw, the screw's pitch and lead are the same; each time the screw's body is rotated by 360 degrees, the screw advances axially by the length of one ridge. Single-start screws have a single starting point where the screw's ridge begins to wrap around the screw.

### SUMMARY

According to exemplary embodiments, a device for delivering fluid to a user includes a reservoir configured to hold the fluid, a plunger received in the reservoir, a threaded multi-start lead screw coupled to the plunger, an actuator coupled to the lead screw and configured to rotate the lead screw, and a processor connected to the actuator and programmed to actuate the actuator to cause a flow of fluid from the reservoir.

Instead of a single-start leadscrew, exemplary embodiments provide a fluid delivery device whose plunger is driven by a multi-start lead screw. A multi-start lead screw allows for more and different types of drive mechanisms to be used in the device, provides more engagement surface for a tube nut connected to the plunger, can be more precise than a single-start lead screw, allows the tube nut to travel farther with each rotation of the lead screw, and assists with variable fill and priming.

In some embodiments, the actuator may comprise a worm drive and a ratchet gear.

In some embodiments, the actuator may comprise an ortho-planar spring and a shape memory alloy (SMA) wire. In some of these embodiments, a finger may be actuated by the SMA wire. A ratchet wheel may interface with the finger. A locking mechanism may be fixed to an internal face of the ortho-planar spring and may include a plurality of teeth configured to mate with the ratchet wheel. The SMA wire may be configured to pull the ratchet wheel tooth-by-tooth past the locking mechanism.

In some embodiments, a restrictor may prevent linear movement of the lead screw as the reservoir is filled, but may permit rotational movement. For example, the restrictor may be an end cap or a bearing surface.

Some embodiments may include a clutch configured to clamp around the lead screw in response to a command signal from the processor indicating that the reservoir is filled. For example, the clutch may include a torsion spring configured to clamp around the leadscrew.

Some embodiments, may include a tube nut connected to the plunger. The tube nut may be configured to be driven along the lead screw when actuated by the actuator.

In some embodiments, the lead screw has two, three, or four starts. In some embodiments, the lead screw specifically uses four starts.

In some embodiments, the actuator is a first drive mechanism configured to rotate the lead screw according to a first increment. A second drive mechanism may also be provided, where the second drive mechanism is configured to rotate the lead screw according to a second increment different from the first increment. In such embodiments, the first drive mechanism may indirectly rotate the lead screw and the second drive mechanism may directly rotate the lead screw so that the first increment is smaller than the second increment.

In some embodiments, the lead screw may be disposed on a side of the plunger. In others, the lead screw may be coaxial with the plunger.

### BRIEF DESCRIPTION OF THE DRAWINGS

FIG. 1 depicts a multi-threaded lead screw according to an exemplary embodiment.
FIG. 2A is an exploded perspective view of a fluid delivery device employing a multi-threaded lead screw according to a first embodiment in which the lead screw is side-actuated.
FIG. 2B is a side view of the fluid delivery device according to the first embodiment showing the plunger in a deployed state.
FIG. 2C is a side view of the fluid delivery device according to the first embodiment showing the plunger in a retracted state.
FIG. 2D is a perspective view of a fluid delivery device employing a multi-threaded lead screw according to a second embodiment in which the lead screw is axial to the plunger, with the plunger in a deployed state.
FIG. 2E is a perspective view of a fluid delivery device employing a multi-threaded lead screw according to the second embodiment in which the lead screw is axial to the plunger, with the plunger in a retracted state.
FIG. 2F is a further perspective view of a fluid delivery device employing a multi-threaded lead screw according to the second embodiment in which the lead screw is axial to the plunger, with the plunger in a deployed state.
FIG. 3A is an exploded perspective view of a fluid delivery device employing a multi-threaded lead screw according to a third embodiment in which a mechanism prevents the ratchet wheel from back-driving.
FIG. 3B is a side view of a fluid delivery device employing a multi-threaded lead screw according to the third embodiment.
FIG. 3C is a perspective view of a fluid delivery device employing a multi-threaded lead screw according to the third embodiment.
FIG. 3D depicts the fluid delivery device of FIGs. 3A-3C with the locking mechanism retracted away from the ratchet wheel.
FIG. 3E depicts the fluid delivery device of FIGs. 3A-3C with the locking mechanism snapped back in place against the ratchet wheel.
FIG. 4A is a perspective view of a fluid delivery device employing a multi-threaded lead screw according to a fourth embodiment.
FIG. 4B is a cross sectional view of the planetary gearbox of FIG. 4A.

### DETAILED DESCRIPTION

Exemplary embodiments provide a fluid delivery device whose plunger is driven by a lead screw, such as a multi-start lead screw.

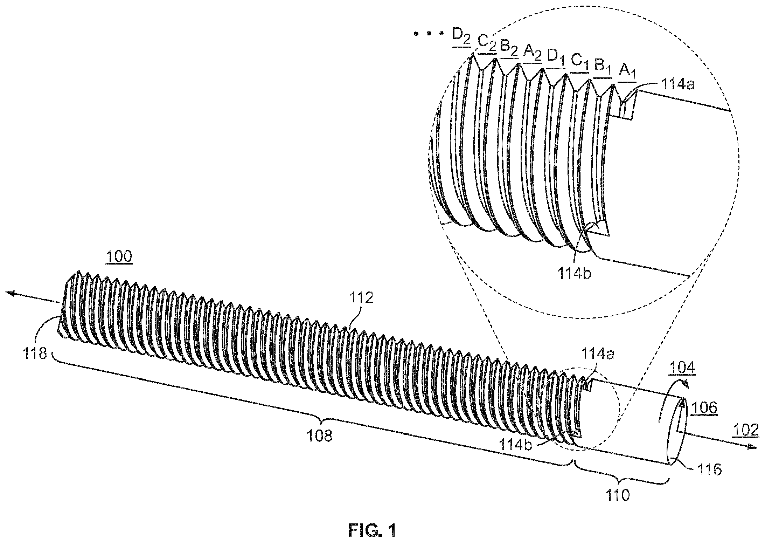

FIG. 1 depicts an exemplary lead screw 100 in accordance with exemplary embodiments. For ease of explanation, an axial direction 102, a circumferential direction 104, and a radial direction 106 are defined. The axial direction 102 extends along a length of the lead screw 100 on its major axis. The circumferential direction 104 extends along the circumference of the lead screw 100, around the outer edge of the lead screw 100. The radial direction 106 extends perpendicular to the axial direction 102, away from the center of the lead screw 100 and towards the screw's outer circumference.

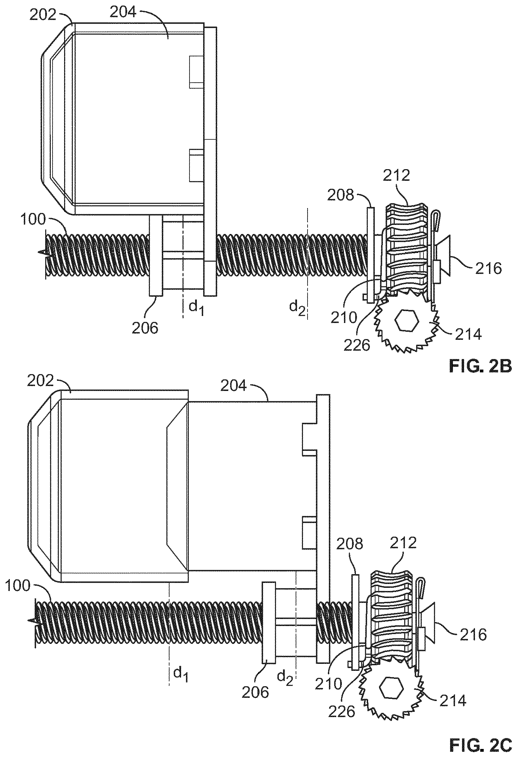

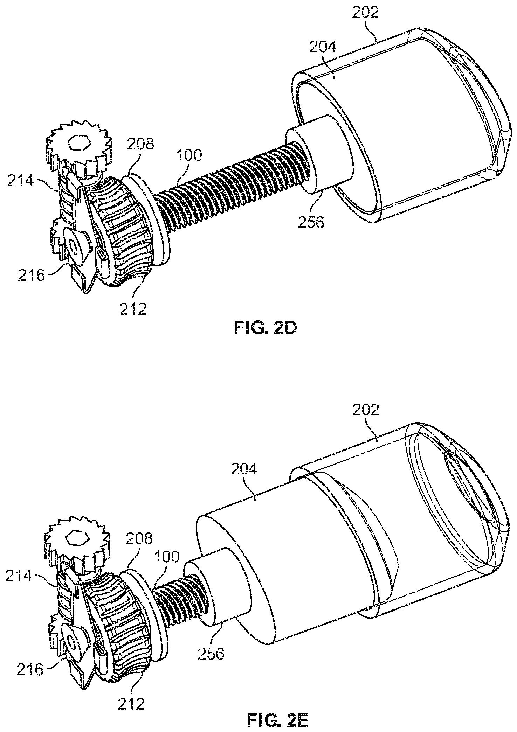

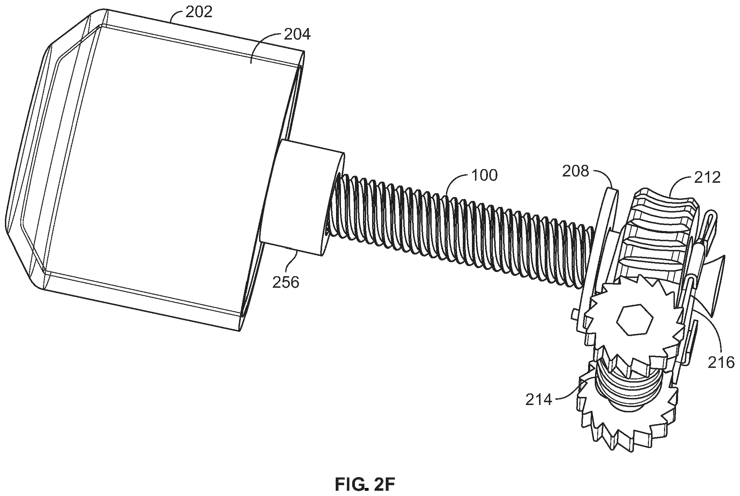

The lead screw 100 extends from a proximal end 116 towards a distal end 118. As used herein, the proximal end 116 refers to the side of the lead screw 100 where, when the plunger of the fluid delivery device is connected closer to the proximal end 116 of the lead screw 100 than the distal end 118, the plunger is more fully retracted from the reservoir as compared to when the plunger is connected closer to the distal end 118 (compare, for instance, FIGs. 2B and 2C).

The lead screw 100 may include a threaded portion 108 which is cut with one or more threads 112 and an unthreaded portion 110 which is not threaded. In exemplary embodiments, the lead screw 100 is multi-threaded in that it includes more than one thread 112 wrapped around the lead screw 100. A separate start 114a, 114b, ... is provided for each thread.

In this embodiment, the lead screw includes four threads 112 and four starts, although only two starts 114a, 114b are visible from the depicted angle. Accordingly, four ridges are wrapped around the lead screw 100. Other embodiments may use two, three, or more than four starts and threads. The specific number of starts and threads will depend on the application, especially the desired diameter of the screw and pitch length.

In FIG. 1, the roots at each rotation of the respective threads are marked as Aᵢ, Bᵢ, Cᵢ, and Dᵢ. Thus, for example, in a single turn of the screw, a nut initially centered at the first location marked A₁ on the proximal end 116 of the screw would advance towards the distal end of the screw 118 by four pitch-lengths (corresponding to the lead-length of the screw) and would then be centered at the second location marked A₂. Consequently, the nut can move further in the axial direction with each rotation of the lead screw 100 as compared to a lead screw with the same pitch but only a single thread 112.

As compared to a lead screw 100 with a single thread having four times the pitch length, (which would advance the above-described nut by the same distance in a single turn), with the multi-start lead screw 100 having four starts there is a much greater amount of surface engagement between the lead screw 100 and the nut. Accordingly, the load is distributed evenly between the threads and the rotation of the nut can be more efficient and precise than with a single-start lead screw. This results in a higher load capacity.

The use of a multi-start lead screw 100 in a fluid delivery device has other advantages over a single start lead screw as well. A multi-start lead screw allows for more and different types of drive mechanisms to be used in the device, provides more engagement surface for a tube nut connected to the plunger, can be more precise than a single-start lead screw, allows the tube nut to travel farther with each rotation of the lead screw, and assists with variable fill and priming as the tube nut can move easily along the length of the leadscrew with each rotation of the screw.

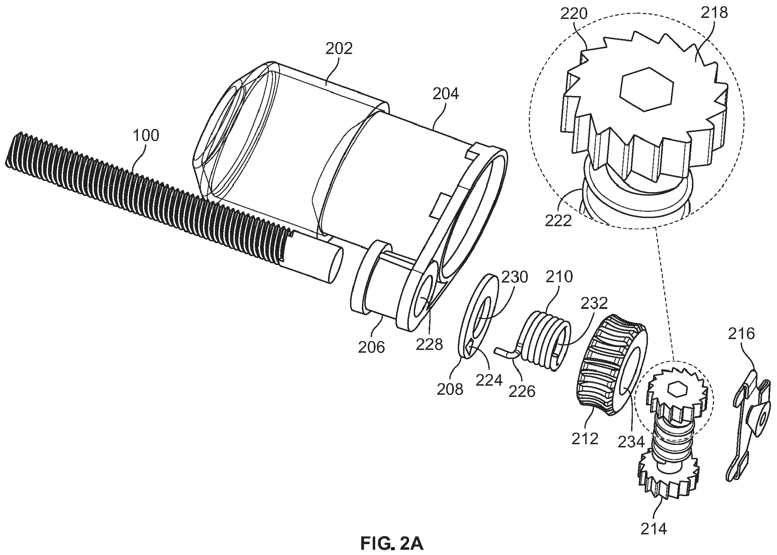

Several different drive mechanisms are now described in connection with FIGs. 2A-2C (side-actuated lead screw), FIGs. 2D-2F (axially-actuated lead screw) and 3A-3C (lead screw with back drive prevention). Each of the described embodiments can be used with a multi-start lead screw 100 as described and shown in FIG. 1, and so repeated descriptions of the multi-start lead screw 100 are omitted for brevity. The multi-start lead screw 100 used in each embodiment may have two, three, four, five, or more starts. The number of starts may be dependent on the application; relevant factors include the chosen pitch length, the diameter of the screw, the type of drive mechanism used, and other factors.

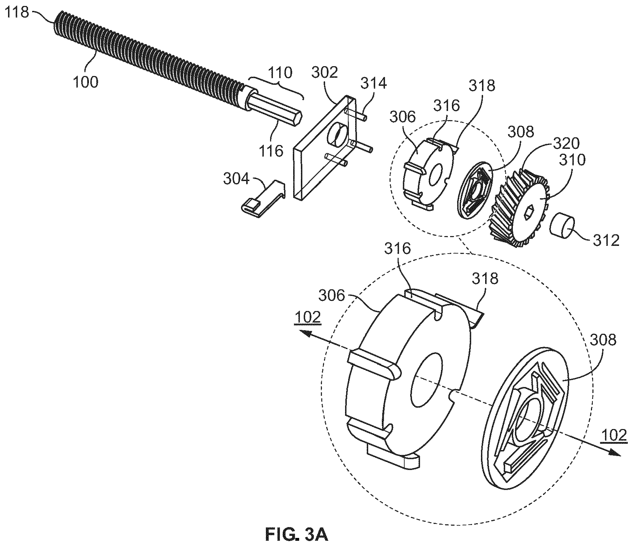

FIGS. 2A-2F and 3A-3C depict various fluid delivery devices using a multi-start lead screw 100 to advance a plunger 204 into a reservoir 202. As used throughout these Figures, like reference numbers are used to refer to like structures, and descriptions of these elements are not repeated between the different Figures for the sake of brevity.

It is contemplated that the fluid delivery device may be used to deliver a drug, such as (but not limited to) insulin (including long-acting and/or rapid-acting insulin analogues) as part of an automated insulin delivery (AID) system; an example of such a system is the Omnipod^{®} system provided by Insulet, Corp. of Acton, MA, USA.

The described embodiments are not limited to drug delivery systems, however, and may be advantageously employed to deliver any suitable fluid from a reservoir. To that end, the reservoir may include a fluid outlet. The reservoir may hold, for example, 0-5 ml of fluid, preferably 1-3 ml, and more preferably about 2 ml. In some embodiments, the reservoir has a capacity between about 0.1 mL to about 5 mL, more specifically between about 1 mL to about 3 mL and in particular between about 1.5 mL to about 2.5 mL. Some advantages of the described fluid delivery devices have been addressed above; additional advantages will be noted on an embodiment-by-embodiment basis in the following description.

FIGs. 2A-2C depict an exemplary fluid delivery device including a side-actuated multi-start lead screw 100. In this case, the side-actuated lead screw 100 is configured so that, when assembled, the lead screw 100 drives a tube nut 206 which is connected with a plunger 204 that can extend a variable distance into a reservoir 202. The lead screw 100 is side-actuated in that it is not co-axial (in the axial direction 102) with the center of the plunger 204, but rather is positioned away from the center of the plunger 204 in the radial direction 106.

The reservoir 202 may be configured to hold a fluid, such as a drug. The plunger 204 may be configured to seat in the reservoir 202 and form a fluid-tight seal. The plunger 204 may be advanced, via the tube nut 206, into (see FIG. 2B) or out of (see FIG. 2C) the reservoir 202. The tube nut 206 may connect the multi-start lead screw 100 to the plunger 204 (e.g., using a collar). Any combination of the tube nut 206, plunger 204, and/or collar may be made integral with each other, or these elements may be maintained as separate pieces.

An actuator may be coupled to the lead screw and configured to rotate the lead screw. The depicted embodiment actuates the multi-start lead screw 100 using a ratcheting worm drive 214 that drives a worm wheel 212. The worm drive 214 includes a worm 222 (also referred to as a worm screw) attached to a ratchet gear 218. The ratchet gear 218 may include a number of ratchet gear teeth 220 that can preferentially advance in one direction to drive the worm 222 forward, but do not allow the worm 222 to rotate in the reverse direction. Thus, when the ratcheting worm drive 214 is engaged it prevents the multi-start lead-screw 100 from back-driving, which could result in an inconsistent or unpredictable amount of fluid being delivered.

The ratcheting worm drive 214 may initially be in an unengaged state. In this state, the reservoir 202 may be empty (e.g., with the tube nut 206 positioned at a first location *d₁* along the length of the threaded portion 108 of the lead screw 100, such that the plunger 204 is fully inserted in the reservoir, in the configuration shown in FIG. 2B) and the lead screw 100 is capable of rotating freely. However, the lead screw 100 may be prevented from shifting linearly (in the axial direction 102) using a portion of pusher 216 which functions as an end cap. The pusher 216 may also be referred to as restrictor 216. As an alternative or in addition to the end cap, a bearing surface may be provided to prevent lateral movement.

As the reservoir 202 is filled, the tube nut 206 that is coupled to the plunger 204 rotates the lead screw 100 in the counterclockwise direction (in the depicted example; one of ordinary skill will recognize that the configuration of the threads in the lead screw 100 can be reversed so that the lead screw 100 rotates in the clockwise direction during filling). In this context, the plunger 204 moves towards the proximal end of the reservoir 202, simultaneously rotating the lead screw 100 in the counterclockwise direction, until the tube nut 206 is positioned at a second position *d₂* that is closer to the proximal end 116 of the lead screw than the first position *d₁.* This rotation allows for a smooth and efficient variable fill.

After the reservoir 202 is filled, the ratcheting worm drive 214 may be engaged with the lead screw 100. In order to engage the ratcheting worm drive 214 with the lead screw 100, the fluid delivery device may apply a clutch mechanism 210. In the depicted embodiment, the clutch mechanism 210 is in the form of a torsion spring that wraps around a clutch opening 232 through which the lead screw 100 can pass. A torsion spring end 226 is configured to extend in the axial direction 102 away from the clutch mechanism 210 and fits within a clutch plate interface 224 (e.g., a hole extending through the clutch plate 208).

In some embodiments, a processor is connected to the actuator and programmed to actuate the actuator, in particular to cause a flow of fluid from the reservoir. The processor may be configured to determine whether, or how much, the reservoir 202 is filled.

The ratcheting worm drive 214 may be operated by one or more shape memory alloy (SMA) wires that alternately rotate pusher 216 about a central axis. For instance, a separate SMA wire may be attached to each of the arms on the opposing sides of the pusher 216, or one arm may be provided with an SMA wire and the other may be attached to a return spring. Such a wire may change size or shape in response to a stimulus; for example, the stimulus may be an electrical current or heat. The application of the electrical current (or heat) may be controlled by the processor. By applying the stimulus to the SMA wire, the ratchet gear 218 may be caused to advance one tooth 220 at a time as it is pushed by pusher 216. This in turn rotates the worm 222 by a small increment, e.g. a first increment, which rotates the worm wheel 212. As the worm wheel 212 rotates, it causes the torsion spring of the clutch mechanism 210 to clamp around the lead screw 100. In some embodiments, the clutch is configured to clamp around the lead screw in response to a command signal from the processor indicating that the reservoir is filled.

The entire system is then in an actuated state, and further activation of the ratcheting worm drive 214 rotates the lead screw 100 in the clockwise direction, driving the tube nut 206 axially along the length of the lead screw 100. This drives the plunger 204 into the reservoir 202 and dispenses the fluid from the reservoir through the fluid delivery interface. Compared to other actuation methods, the ratcheting worm drive 214 has a very large mechanical advantage which will turn the lead screw 100 in extremely small rotational increments. This allows for a very precise amount of fluid to be dispensed.

FIGs. 2B and 2C show the fluid delivery device in an assembled state. To assemble the device, the unthreaded side 110 of the multi-start lead screw 100 may be inserted through a tube nut opening 228 of the tube nut 206. By rotating the lead screw 100, the tube nut 206 may be advanced part of the way along the threaded side 108 of the lead screw 100, exposing the unthreaded end 110 through the opposite side of the tube nut 206. The lead screw 100 may then be advanced through a clutch plate opening 230 in the clutch plate 208.

The clutch mechanism 210 may be engaged with the clutch plate 208 (such as by inserting the torsion spring end 226 into the clutch plate interface 224. The lead screw 100 may be inserted through a clutch hole 232 in the clutch 210. The worm wheel 212 may be slid over the clutch mechanism 210 and the lead screw 100 (which is internal to the clutch mechanism 210, in this embodiment). The lead screw 100 may proceed through the clutch hole 232 and may attach to or abut the end cap portion of pusher 216 (or a similarly-situated bearing surface). The ratcheting worm drive 214 may then be brought into contact with the worm wheel 212 (or vice versa).

Rotating the lead screw 100 with the ratcheting drive mechanism 214 may cause the tube nut to advance along threaded part

Other drive mechanisms could be used as an alternative to, or in addition to, the ratcheting drive mechanism 214. For example, the described embodiments could be used with the ratcheting drive mechanism 214 and a second drive mechanism, or two or more other types of drive mechanism. This would allow the fluid delivery device to switch between providing a regular pulse of fluid from the reservoir 202 versus a bolus of fluid depending on how or the degree to which the lead screw 100 is rotated. For example, the worm drive 214 allows for extremely small rotational increments of the lead screw 100, but if coupled with a drive that rotates the lead screw directly or in larger increments, e.g. a second increment, more fluid can be delivered in a shorter period of time. In some embodiments, the fluid delivery device comprises a first drive mechanism configured to rotate the lead screw according to a first increment, and further comprising a second drive mechanism configured to rotate the lead screw according to a second increment different from the first increment. In some embodiments, the fluid delivery device comprises a first drive mechanism configured to rotate the lead screw according to a first speed (or rate of rotation), and further comprising a second drive mechanism configured to rotate the lead screw according to a second speed or rate of rotation different from the first speed (or rate of rotation).

Side-actuated lead-screws of the type shown in the first embodiment have the advantage of space savings: they can be accommodated in less vertical space than other configurations, which means that the size of the reservoir 202 (and hence the amount of fluid accommodated) can be greater, and/or the overall size of the pumping system can be reduced.

An example of a lead screw 100 that is not side-actuated (but rather mounted axial to the plunger 204) is depicted in FIGs. 2D-2F.

In this embodiment, instead of the tube nut 206, the lead screw 100 may be threaded through a plunger interface 256 that may be integral with or attached to the plunger 204. In this example, the lead screw 100 extends into and out of the plunger interface 256 and/or the plunger 204. Alternatively, the lead screw 100 might be permitted to extend through the back of the device, in the vicinity of the pusher 216.

This embodiment prevents a moment being applied to the plunger 204 through side actuation, will require less force to drive the plunger 204, and makes manufacturing of the components less costly and easier.

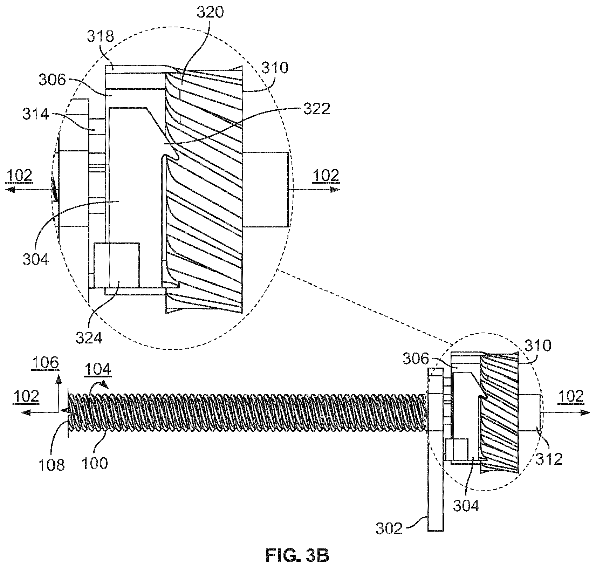

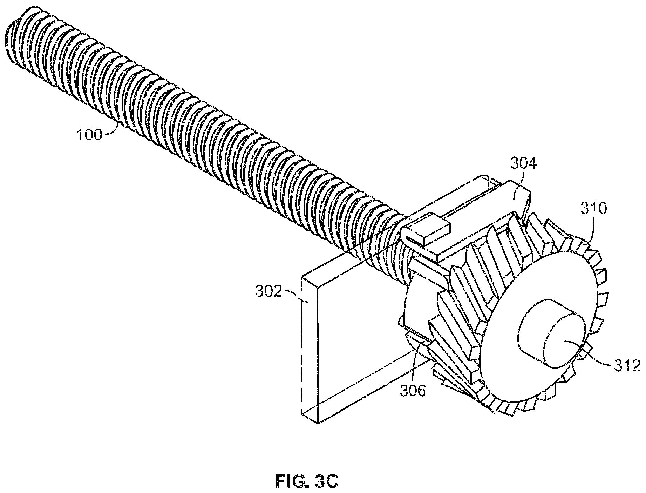

FIGs. 3A-3C depict another embodiment that can be used to prevent the lead screw 100 from back-driving or over-dispensing the fluid. In this embodiment, the lead screw 100 may be actuated using an ortho-planar spring 308 in conjunction with a shape memory allow (SMA) wire. FIG. 3A shows an exploded view of the multi-start lead screw 100 and actuation mechanism, with an inset showing how the ortho-planar spring 308 interacts with a locking mechanism 306. FIG. 3B shows the same system in an assembled state, with an inset highlighting the interaction between a ratchet wheel 310 and finger 304. FIG. 3C is a perspective view of the assembled system. For ease of discussion, the reservoir 202 and plunger 204 are omitted from these depictions, but could be provided in a similar configuration to either of the embodiments discussed above.

In the third embodiment, a specially-shaped base plate 302 includes a hole through which the lead screw 100 can pass and a set of base plate rails 314. The base plate rails 314 fit into rail grooves 316 in a specially-shaped locking mechanism 306. The rails 314 and grooves are configured so that the locking mechanism 306 can slide in the axial direction 102 along the rails 314.

The locking mechanism 306, shown in more detail in the inset to FIG. 3A, is a disc-shaped element that includes the grooves 316 for slotting in the rails 314 of the base plate 302, a central opening that can accommodate the lead screw 100, and locking mechanism teeth 318 configured to interact, in particular mate, with a ratchet wheel 310, as discussed in more detail below.

The locking mechanism 306 is fixed to an internal face of an ortho-planar spring 308. The ortho-planar spring 308 is a compliant mechanism that allows for a linear actuation or extension and the ability to snap back into a flat planar configuration. It may be used to counter an SMA wire pulling in one direction. The ortho-planar spring 308 is connected to both the locking mechanism 306 and the ratchet wheel 310. When the ortho-planar spring 308 is in its flat, planar configuration (or collapsed configuration), the locking mechanism 306 and ratchet wheel 310 are in close proximity to each other. When the ortho-planar spring 308 is in its extended position, the locking mechanism 306 is extended away from the ratchet wheel 310.

A finger 304 (see inset of FIG. 3B) includes a finger tooth 322 and a u-shaped connector or crimp 324 for connection to an SMA wire. The tooth 322 and the teeth 320 of the ratchet wheel 320 are shaped in a complimentary manner so that, when the finger is actuated by the SMA wire, it pulls the ratchet wheel 320 by a single ratchet wheel tooth 320 past the locking mechanism teeth 318, in particular tooth-by-tooth.

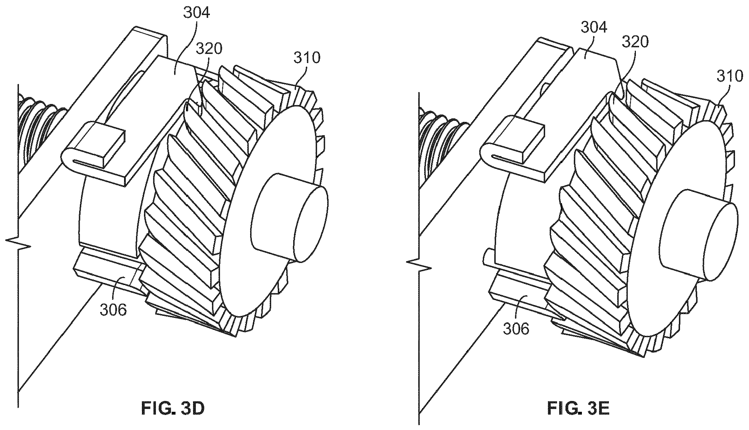

During this actuation, the locking mechanism 306 in combination with the ortho-planar spring 308 causes these components to slide along the rails 314 of the base plate 302. This separates the locking mechanism teeth 318 from the ratchet wheel 310 (shown in FIG. 3D), allowing the ratchet wheel 310 to rotate. After the actuation is complete, the locking mechanism 306 snaps back against the ratchet wheel 310, assisted by the ortho-planar spring 308 (show in FIG. 3E), to hold the ratchet wheel from back-driving or over dispensing. The ratchet wheel 310 and lead screw 100 are connected to each other via a hexagonal interface, and rotation of the ratchet wheel 310 causes rotation of the lead screw 100.

FIGs. 3D-3E also show the action of the SMA wire. The SMA wire may pull the finger 304 downwards, away from the top of the base plate 302 (namely, towards the bottom of FIG. 3B, or down and to the left in FIGs. 3D and 3E). FIG. 3D shows the finger 304 after it has been pulled downwards by the SMA wire, whereas FIG. 3E shows the finger 304 having returned to its original position.

The fluid delivery system is terminated by an end cap 312, similar in functionality to the end cap portion of pusher 216 discussed above, or a bearing surface.

In this embodiment, the lead-screw is directly driven through the action of the ratchet wheel 310. Alternatively or in addition, this embodiment can be combined with gearing or another mechanism that increases the mechanical advantage of the system.

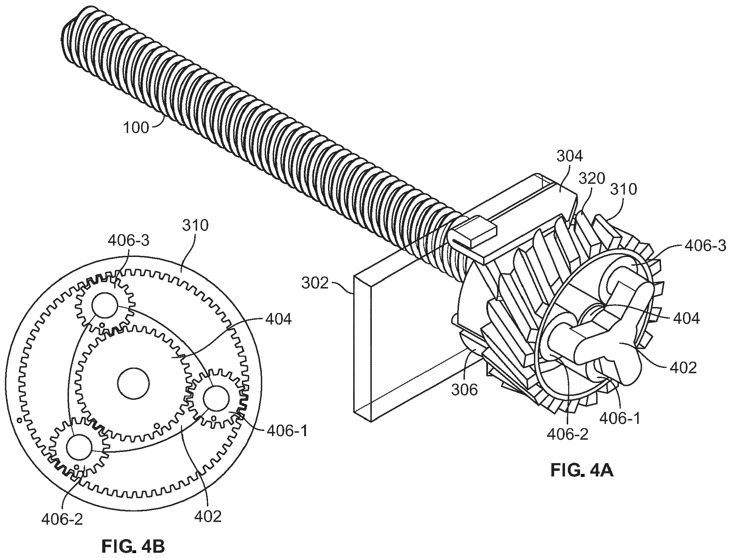

FIGs. 4A-4B depict a fourth embodiment that is similar in many respects to the embodiment depicted in FIGs 3A-3E. For the sake of brevity, further description of repeated elements is omitted.

In the fourth embodiment, a planetary gearbox is provided inside the ratchet wheel 310 to provide a gear reduction. The planetary gearbox includes a central sun gear 404 and a number of planet gears 406-1, 406-2, 406-3 that orbit around the sun gear 404. Although FIG. 4A depicts three planet gears, it is understood that more or fewer planet gears may be employed.

The ratchet wheel 310 serves as a ring gear which is the input for the planetary gearbox. The sun gear 404 is fixed in place and is mechanically connected to a carrier 402 that serves as the output of the gearbox. The carrier 402 is attached to the planet gears 406-1, 406-2, 406-3 and is mounted to the end of the leadscrew 100. The relationship between these components is shown in more detail in FIG. 4B.

As the SMA wire is pulled to actuate the finger 304, the ratchet wheel 310 turns by one tooth 320. Therefore, the output to the leadscrew 100 is reduced as defined by the number of teeth 320 and number of planet gears 406 involved in the system.

By way of example (and without limiting the invention), the ratchet wheel 310 may have 45 teeth, the sun gear 404 may have 27 teeth, and the planet gears 406 may each have 9 teeth. This configuration would give a reduction of 1.6:1 for the ratchet wheel to the leadscrew. If more mechanical advantage is required, the number of teeth between the ratchet wheel, sun, and planet gears can change. Alternatively or in addition, planetary gear sets may be stacked to achieve a much larger gear reduction. While the present disclosure has been illustrated and described in detail in the drawings and foregoing description, the same is to be considered as illustrative and not restrictive in character, it being understood that only the certain embodiments have been shown and described and that all changes, alternatives, modifications and equivalents that come within the spirit of the disclosure are desired to be protected.

It should be understood that while the use of words such as preferable, preferably, preferred or more preferred utilized in the description above indicate that the feature so described may be more desirable, it nonetheless may not be necessary and embodiments lacking the same may be contemplated as within the scope of the present disclosure, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. A device for delivering fluid to a user, comprising:
a reservoir configured to hold the fluid;
a plunger received in the reservoir;
a threaded multi-start lead screw coupled to the plunger;
an actuator coupled to the lead screw and configured to rotate the lead screw; and
a processor connected to the actuator and programmed to actuate the actuator to cause a flow of fluid from the reservoir.

2. The device of claim 1, wherein the actuator comprises a worm drive and a ratchet gear.

3. The device of claim 1 or 2, wherein the actuator comprises an ortho-planar spring and a shape memory alloy (SMA) wire.

4. The device of claim 3, further comprising a finger actuated by the SMA wire, a ratchet wheel that interfaces with the finger, and a locking mechanism fixed to an internal face of the ortho-planar spring and having a plurality of teeth configured to mate with the ratchet wheel, wherein the SMA wire is configured to pull the ratchet wheel tooth-by-tooth past the locking mechanism.

5. The device of any one of claims 1 to 4, further comprising a restrictor that prevents linear movement of the lead screw as the reservoir is filled but permits rotational movement.

6. The device of claim 5, wherein the restrictor comprises an end cap or a bearing surface.

7. The device of any one of claims 1 to 6, further comprising a clutch configured to clamp around the lead screw in response to a command signal from the processor indicating that the reservoir is filled.

8. The device of claim 7, wherein the clutch comprises a torsion spring configured to clamp around the leadscrew.

9. The device of any one of claims 1 to 8, further comprising a tube nut connected to the plunger, the tube nut configured to be driven along the lead screw when actuated by the actuator.

10. The device of any one of claims 1 to 9, wherein the lead screw has two, three, or four starts.

11. The device of claims 1 to 10, wherein the actuator is a first drive mechanism configured to rotate the lead screw according to a first increment, and further comprising a second drive mechanism configured to rotate the lead screw according to a second increment different from the first increment.

12. The device of claim 11, wherein the first drive mechanism indirectly rotates the lead screw and the second drive mechanism directly rotates the lead screw so that the first increment is smaller than the second increment.

13. The device of any one of claims 1 to 12, wherein the lead screw is disposed on a side of the plunger.

14. The device of any one of claims 1 to 12, wherein the lead screw is coaxial with the plunger.
